# EUROPÄISCHE PATENTSCHRIFT

(11) **EP 4 011 894 B1**
(45) Veröffentlichungstag und Bekanntmachung des Hinweises auf die Patenterteilung: **31.05.2023**
(21) Anmeldenummer: 20212754.4
(22) Anmeldetag: 09.12.2020
(51) Int. Cl.: C07F 15/00, C07C 51/14, C07C 53/126

(54) **PLATIN-KOMPLEXE MIT BINAPHTHYL-DIPHOSPHIN-LIGANDEN FÜR DIE KATALYSE DER HYDROXYCARBONYLIERUNG ETHYLENISCH UNGESÄTTIGTER VERBINDUNGEN**
PLATINUM COMPLEXES WITH BINAPHTHYL DIPHOSPHINE LIGANDS FOR THE CATALYSIS OF HYDROXY CARBONYLATION OF ETHYLENICALLY UNSATURATED COMPOUNDS
COMPLEXE DU PLATINE COMPRENANT DES LIGANDS BINAPHTHYL DIPHOSPHINE POUR LA CATALYSE DE L'ALCOXYCARBONYLATION DES COMPOSÉS ÉTHYLÉNIQUEMENT NON SATURÉS

(43) Veröffentlichungstag der Anmeldung: 15.06.2022
(73) Patentinhaber: Evonik Operations GmbH, 45128 Essen (DE)
(72) Erfinder: SCHNEIDER, Carolin, 40789 Monheim am Rhein (DE); JACKSTELL, Ralf, 18106 Rostock (DE); BELLER, Matthias, 18211 Ostseebad Nienhagen (DE); FRANKE, Robert, 45772 Marl (DE)
(74) Vertreter: Evonik Patent Association

(56) Entgegenhaltungen:
- DE-A1- 10 342 760
- YANG JI ET AL: "A general platinum-catalyzed alkoxycarbonylation of olefins", CHEMICAL COMMUNICATIONS, Bd. 56, Nr. 39, 14. Mai 2020 (2020-05-14), Seiten 5235-5238, XP055798750, ISSN: 1359-7345, DOI: 10.1039/D0CC00650E

## Beschreibung

Die vorliegende Erfindung betrifft Platin-Komplexe mit Binaphthyl-Diphosphin-Liganden für die Katalyse der Hydroxycarbonylierung ethylenisch ungesättigter Verbindungen.

Die Hydroxycarbonylierung ethylenisch ungesättigter Verbindungen ist ein Prozess mit steigender Bedeutung. Unter einer Hydroxycarbonylierung versteht man die direkte Umsetzung von ethylenisch ungesättigten Verbindungen, wie Olefinen, mit Kohlenmonoxid in Gegenwart eines Metalls bzw. eines Metallkomplexes und eines Liganden zu den entsprechenden Säuren:

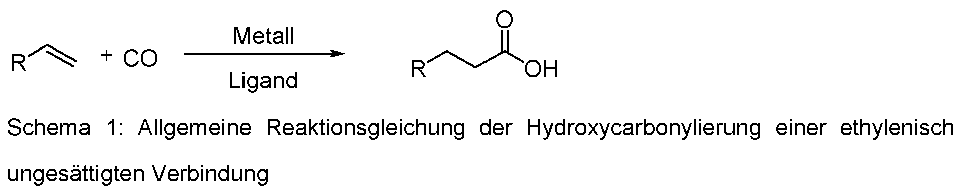

In EP 3388414 A1 wird ein Verfahren zur Hydroformylierung von Cyclooctadien (COD) unter Einsatz von 4-([1,1':3',1"-Terphenyl]-2'-yloxy)-S-dinaphtho[2,1-d:1',2'-f][1,3,2]dioxa-phosphepin beschrieben. Weitere Reaktionen beinhaltend Alkoxycarbonylierung von Alkenen katalysiert durch Platinum-Komplexe sind auch bekannt (Chemical Communcations, Bd.56, Nr 39, Seiten 5235-5238). DE10342760 offenbart Pnicogenverbindungen und Katalysatoren die solche Verbindungen beinhalten zur Hydroformylierung.

Ein Nachteil von Palladium ist dessen hoher Preis.

Die technische Aufgabe, welche der vorliegenden Erfindung zugrunde lag, ist die Bereitstellung neuer Komplexe, welche ein Metall als Zentralatom aufweisen, welches einen niedrigeren Preis als Palladium hat. Mit den Komplexen sollen zudem bei Hydroxycarbonylierungen gute Umsätze erzielt werden.

Diese Aufgabe wird gelöst durch einen Komplex nach Anspruch 1.

Komplex umfassend Pt und eine Verbindung gemäß Formel (**I**) 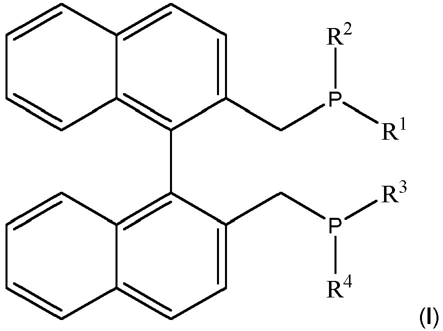 wobei
R¹, R², R³, R⁴ jeweils unabhängig voneinander ausgewählt sind aus -(C₁-C₁₂)-Alkyl, -(C₆-C₂₀)-Heteroaryl.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Der Begriff (C₆-C₂₀)-Heteroaryl umfasst mono- oder polycyclische aromatische Kohlenwasserstoffreste mit 6 bis 20 Kohlenstoffatomen, wobei eins oder mehrere der Kohlenstoffatome durch Heteroatome ersetzt sind. Bevorzugte Heteroatome sind N, O und S. Die (C₃-C₂₀)-Heteroarylgruppen weisen 6 bis 20, bevorzugt 6 bis 14, besonders bevorzugt 6 bis 10 Ringatome auf. Somit ist beispielsweise Pyridyl im Rahmen dieser Erfindung ein C₆-Heteroarylrest.

Geeignete (C₆-C₂₀)-Heteroarylgruppen mit mindestens sechs Ringatomen sind insbesondere Pyridyl, Pyridazinyl, Pyrimidyl, Pyrazinyl, Benzofuranyl, Indolyl, Isoindolyl.

In einer Ausführungsform stehen mindestens zwei der Reste R¹, R², R³, R⁴ für einen -(C₆-C₂₀)-Heteroarylrest mit mindestens sechs Ringatomen.

In einer Ausführungsform stehen die Reste R¹ und R³ jeweils für einen -(C₆-C₂₀)-Heteroarylrest mit mindestens sechs Ringatomen.

In einer Ausführungsform stehen die Reste R¹ und R³ jeweils für 2-Pyridyl.

In einer Ausführungsform stehen R² und R⁴ für -(C₁-C₁₂)-Alkyl.

In einer Ausführungsform stehen R² und R⁴ für *tert*-Butyl.

In einer Ausführungsform weist die Verbindung (**I**) die Struktur (**1**) auf:

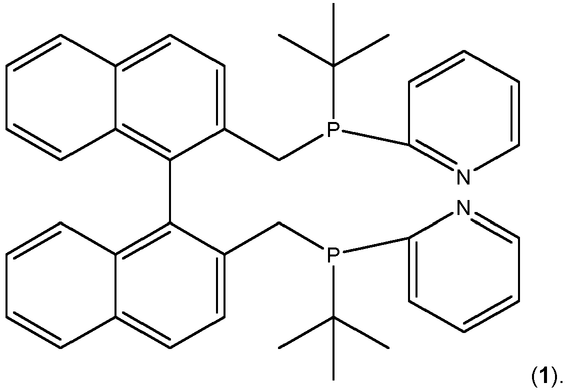

Die Erfindung betrifft weiterhin die Verwendung eines erfindungsgemäßen Komplexes zur Katalyse einer Hydroxycarbonylierungsreaktion.

Verfahren umfassend die Verfahrensschritte:
a) Vorlegen einer ethylenisch ungesättigten Verbindung;
b) Zugabe eines wie zuvor beschriebenen Komplexes, oder eine Verbindung gemäß Formel (**I**) 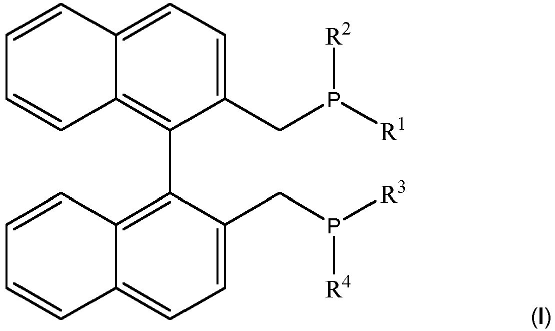 wobei
   R¹, R², R³, R⁴ jeweils unabhängig voneinander ausgewählt sind aus -(C₁-C₁₂)-Alkyl, -(C₆-C₂₀)-Heteroaryl und
   einer Substanz, welche Pt umfasst;
c) Zugabe einer Säure;
d) Zuführen von CO;
e) Erwärmen des Reaktionsgemisches aus a) bis d), wobei die ethylenisch ungesättigte Verbindung zu einer Carbonsäure umgesetzt wird.

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis c) vorgelegt wurden. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Die in dem erfindungsgemäßen Verfahren als Edukt eingesetzten ethylenisch ungesättigten Verbindungen enthalten eine oder mehrere Kohlenstoff-Kohlenstoff-Doppelbindungen. Diese Verbindungen werden auch im Folgenden zur Vereinfachung als Olefine bezeichnet. Die Doppelbindungen können terminal oder intern sein.

In einer Variante des Verfahrens umfasst die ethylenisch ungesättigte Verbindung keine weiteren funktionellen Gruppen außer Kohlenstoff-Kohlenstoff-Doppelbindungen.

In einer Variante des Verfahrens ist die ethylenisch ungesättigte Verbindung ausgewählt aus: Ethen, Propen, 1-Buten, *cis-* und/oder trans-2-Buten, iso-Buten, 1,3-Butadien, 1-Penten, *cis-* und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

In einer Variante des Verfahrens ist die Säure in Verfahrensschritt c) ausgewählt aus: Essigsäure, Perchlorsäure, Schwefelsäure, Phosphorsäure, Methylphosphonsäure, Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure, Dodecylsulfonsäure, Camphersulfonsäure.

In einer Variante des Verfahrens ist die Säure in Verfahrensschritt c) Essigsäure (AcOH).

In einer Variante des Verfahrens ist die Substanz, welche Pt umfasst ausgewählt aus: Platindichlorid (PtCl₂), Platin(II)-acetylacetonat [Pt(acac)₂], Platin(II)-acetat [Pt(OAc)₂, Dichloro(1,5-cyclooctadiene)platin(II) [Pt(cod)₂Cl₂], Bis(dibenzylideneaceton)platin [Pt(dba)₂], Bis(acetonitrile)dichloropatin(II) [Pt(CH₃CN)₂Cl₂], Platin(cinnamyl)dichlorid [Pt(cinnamyl)Cl₂],

In einer Variante des Verfahrens ist die Substanz, welche Pt umfasst ausgewählt aus: Platindichlorid (PtCl₂), Platin(II)-acetylacetonat [Pt(acac)₂], Platin(II)-acetat [Pt(OAc)₂].

CO wird in Schritt d) vorzugsweise bei einem CO-Partialdruck zwischen 0,1 und 10 MPa (1 bis 100 bar), bevorzugt zwischen 1 und 8 MPa (10 bis 80 bar), besonders bevorzugt zwischen 2 und 6 MPa (20 bis 60 bar) zugeführt.

Die Reaktionsmischung wird in Schritt e) des erfindungsgemäßen Verfahrens vorzugsweise auf eine Temperatur im Bereich von 60 °C bis 160 °C, bevorzugt von 80 °C bis 140 °C, besonders bevorzugt von 80 °C bis 120 °C erwärmt, um die ethylenisch ungesättigte Verbindung zu einer Säure umzusetzen.

Im Folgenden soll die Erfindung anhand eines Ausführungsbeispieles näher erläutert werden.

### Umsetzung von 1-Octen zur Säure

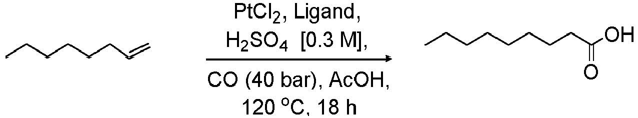

Reaktionsbedingungen: 1-Octen (1.0 mmol), PtCl₂ (0.01 mmol, 1.0 mol%), Ligand: bidentater Phosphinligand (0.022 mmol, 2.2 mol%), Schwefelsäure [0.3 M] 0.5 mL, AcOH (1.5 mL), Druck (CO): 40 bar (=4000kPa), Temperatur: 120 °C, Reaktionszeit: 18 h.

Die Reaktion wurde in einem erfindungsgemäßen Verfahren mit dem Ligand (1), sowie als Vergleichsversuch mit dem Liganden (2) durchgeführt:

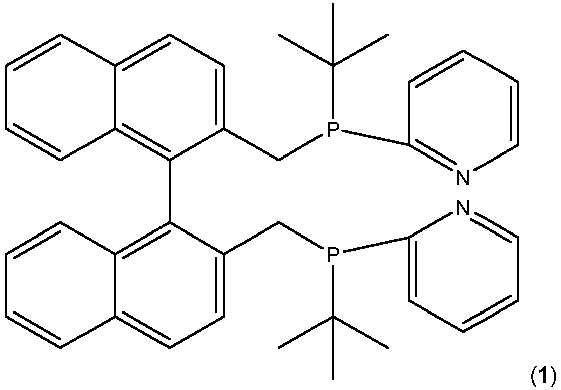

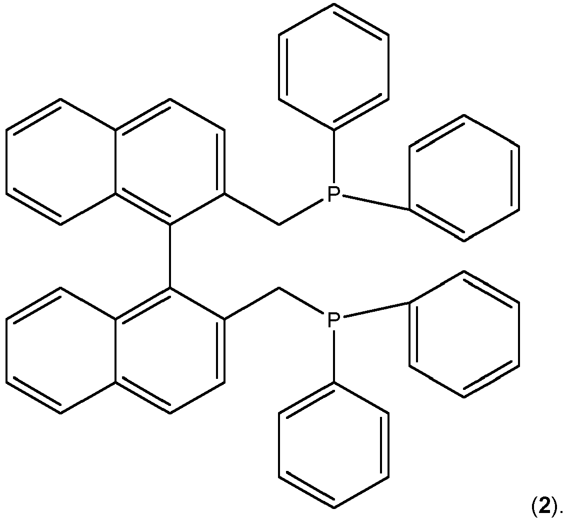

Das erfindungsgemäße Verfahren mit Ligand (1) lieferte hierbei eine Ausbeute von 50%. In dem Vergleichsversuch mit Ligand (2) konnte hingegen nur eine Ausbeute von 26% erzielt werden.

Der Preis von Pt liegt unter dem von Pd. Die Aufgabe wird somit durch einen erfindungsgemäßen Komplex gelöst.

## Patentansprüche

1. Komplex umfassend Pt und eine Verbindung gemäß Formel (**I**) 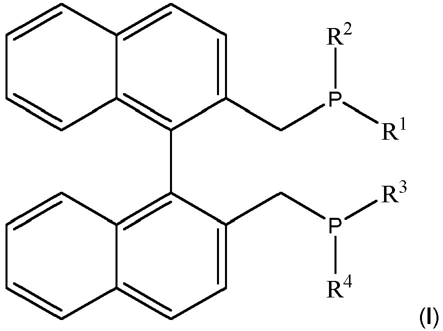 wobei
R¹, R², R³, R⁴ jeweils unabhängig voneinander ausgewählt sind aus -(C₁-C₁₂)-Alkyl,-(C₆-C₂₀)-Heteroaryl.

2. Komplex nach Anspruch 1,
wobei mindestens zwei der Reste R¹, R², R³, R⁴ für einen -(C₆-C₂₀)-Heteroarylrest mit mindestens sechs Ringatomen stehen.

3. Komplex nach einem der Ansprüche 1 bis 2,
wobei die Reste R¹ und R³ jeweils für einen -(C₆-C₂₀)-Heteroarylrest mit mindestens sechs Ringatomen stehen.

4. Komplex nach einem der Ansprüche 1 bis 3,
wobei die Reste R¹ und R³ jeweils für 2-Pyridyl stehen.

5. Komplex nach einem der Ansprüche 1 bis 4,
wobei R² und R⁴ für -(C₁-C₁₂)-Alkyl stehen.

6. Komplex nach einem der Ansprüche 1 bis 5,
wobei R² und R⁴ für tert-Butyl stehen.

7. Komplex nach einem der Ansprüche 1 bis 6,
wobei die Verbindung (**I**) die Struktur (**1**) aufweist:

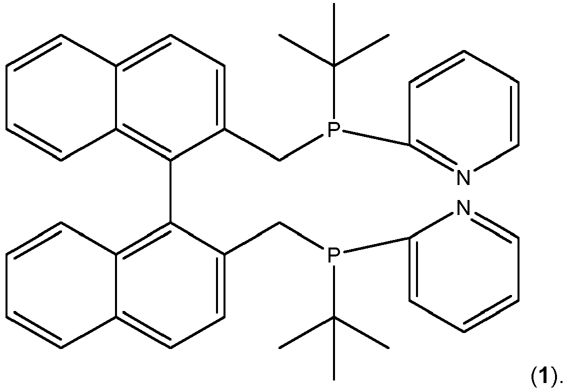

8. Verfahren umfassend die Verfahrensschritte:
a) Vorlegen einer ethylenisch ungesättigten Verbindung;
b) Zugabe eines Komplexes nach einem der Ansprüche 1 bis 7, oder eine Verbindung gemäß Formel (**I**) 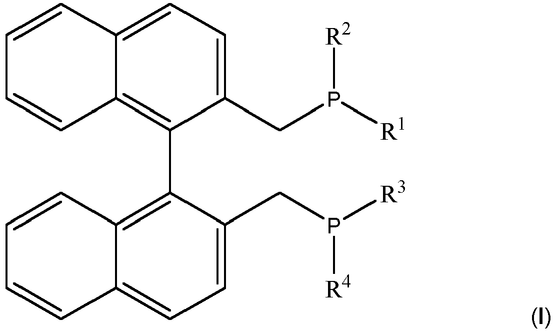 wobei
R¹, R², R³, R⁴ jeweils unabhängig voneinander ausgewählt sind aus -(C₁-C₁₂)-Alkyl,-(C₆-C₂₀)-Heteroaryl und
einer Substanz, welche Pt umfasst;
c) Zugabe einer Säure;
d) Zuführen von CO;
e) Erwärmen des Reaktionsgemisches aus a) bis d), wobei die ethylenisch ungesättigte Verbindung zu einer Carbonsäure umgesetzt wird.

9. Verfahren nach Anspruch 8,
wobei die ethylenisch ungesättigte Verbindung ausgewählt ist aus: Ethen, Propen, 1-Buten, *cis-* und/oder trans-2-Buten, iso-Buten, 1,3-Butadien, 1-Penten, *cis-* und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

10. Verfahren nach einem der Ansprüche 8 oder 9,
wobei die Säuere in Verfahrensschritt c) ausgewählt ist aus: Essigsäure, Perchlorsäure, Schwefelsäure, Phosphorsäure, Methylphosphonsäure, Methansulfonsäure, Trifluormethansulfonsäure, tert-Butansulfonsäure, p-Toluolsulfonsäure, 2-Hydroxypropan-2-sulfonsäure, 2,4,6-Trimethylbenzolsulfonsäure, Dodecylsulfonsäure, Camphersulfonsäure.

11. Verfahren nach einem der Ansprüche 8 bis 10,
wobei die Säure in Verfahrensschritt c) Essigsäure ist.

12. Verfahren nach einem der Ansprüche 8 bis 11,
wobei die Substanz, welche Pt umfasst ausgewählt ist aus: Platindichlorid (PtCl₂), Platin(II)-acetylacetonat [Pt(acac)₂], Platin(II)-acetat [Pt(OAc)₂], Dichloro(1,5-cyclooctadiene)platin(II) [Pt(cod)₂Cl₂], Bis(dibenzylideneaceton)platin [Pt(dba)₂], Bis(acetonitrile)dichloropatin(II) [Pt(CH₃CN)₂Cl₂], Platin(cinnamyl)dichlorid [Pt(cinnamyl)Cl₂].

13. Verfahren nach einem der Ansprüche 8 bis 12,
wobei die Substanz, welche Pt umfasst ausgewählt ist aus: Platindichlorid (PtCl₂), Platin(II)-acetylacetonat [Pt(acac)₂], Platin(II)-acetat [Pt(OAc)₂].

## Claims

1. Complex comprising Pt and a compound of formula (I) 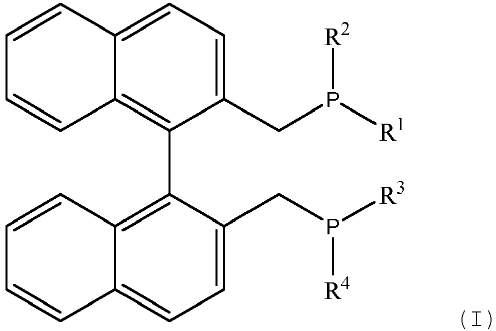 where
R¹, R², R³, R⁴ are each independently selected from -(C₁-C₁₂)-alkyl, -(C₆-C₂₀)-heteroaryl.

2. Complex according to Claim 1,
wherein at least two of the R¹, R², R³, R⁴ radicals are a -(C₆-C₂₀)-heteroaryl radical having at least six ring atoms.

3. Complex according to any of Claims 1 to 2,
wherein the R¹ and R³ radicals are each a -(C₆-C₂₀)-heteroaryl radical having at least six ring atoms.

4. Complex according to any of Claims 1 to 3,
wherein the R¹ and R³ radicals are each 2-pyridyl.

5. Complex according to any of Claims 1 to 4,
wherein R² and R⁴ are -(C₁-C₁₂)-alkyl.

6. Complex according to any of Claims 1 to 5,
wherein R² and R⁴ are tert-butyl.

7. Complex according to any of Claims 1 to 6,
wherein the compound (I) has the structure (1):

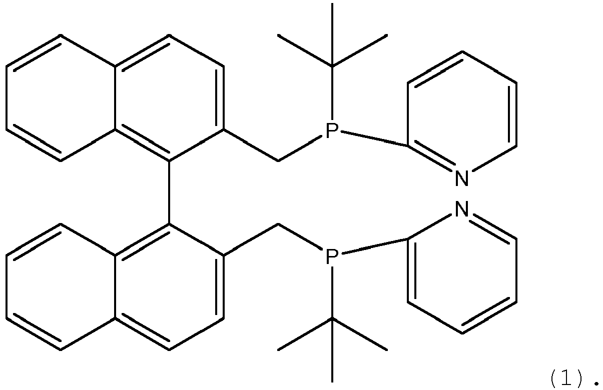

8. Process comprising the process steps of:
a) initially charging an ethylenically unsaturated compound;
b) adding a complex according to any of Claims 1 to 7, or
a compound of formula (I) 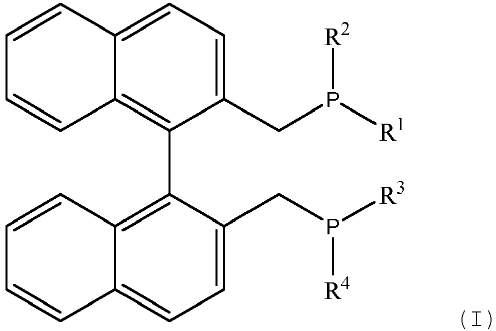
where
R¹, R², R³, R⁴ are each independently selected from -(C₁-C₁₂)-alkyl, -(C₆-C₂₀)-heteroaryl
and
a substance comprising Pt;
c) adding an acid;
d) feeding in CO;
e) heating the reaction mixture from a) to d), with conversion of the ethylenically unsaturated compound to a carboxylic acid.

9. Process according to Claim 8,
wherein the ethylenically unsaturated compound is selected from: ethene, propene, 1-butene, *cis-* and/or trans-2-butene, isobutene, 1,3-butadiene, 1-pentene, *cis-* and/or trans-2-pentene, 2-methyl-1-butene, 3-methyl-1-butene, 2-methyl-2-butene, hexene, tetramethylethylene, heptene, 1-octene, 2-octene, di-n-butene, or mixtures thereof.

10. Process according to either of Claims 8 and 9,
wherein the acid in process step c) is selected from: acetic acid, perchloric acid, sulfuric acid, phosphoric acid, methylphosphonic acid, methanesulfonic acid, trifluoromethanesulfonic acid, tert-butanesulfonic acid, p-toluenesulfonic acid, 2-hydroxypropane-2-sulfonic acid, 2,4,6-trimethylbenzenesulfonic acid, dodecylsulfonic acid, camphorsulfonic acid.

11. Process according to any of Claims 8 to 10,
wherein the acid in process step c) is acetic acid.

12. Process according to any of Claims 8 to 11,
wherein the substance comprising Pt is selected from: platinum dichloride (PtCl₂), platinum(II) acetylacetonate [Pt(acac)₂], platinum(II) acetate [Pt(OAc)₂], dichloro(1,5-cyclooctadiene)platinum(II) [Pt(cod)₂Cl₂], bis(dibenzylideneacetone)platinum [Pt(dba)₂], bis(acetonitrile)dichloroplatinum(II) [Pt(CH₃CN)₂Cl₂], (cinnamyl)platinum dichloride [Pt(cinnamyl)Cl₂].

13. Process according to any of Claims 8 to 12,
wherein the substance comprising Pt is selected from: platinum dichloride (PtCl₂), platinum(II) acetylacetonate [Pt(acac)₂], platinum(II) acetate [Pt(OAc)₂].

## Revendications

1. Complexe comprenant du Pt et un composé selon la formule (I) 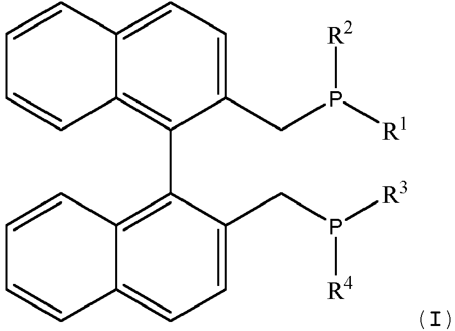 R¹, R², R³, R⁴ étant chacun indépendamment choisis parmi -(C₁-C₁₂)-alkyle, -(C₆-C₂₀)-hétéroaryle.

2. Complexe selon la revendication 1,
au moins deux des radicaux R¹, R², R³, R⁴ représentant un radical -(C₆-C₂₀)-hétéroaryle comportant au moins six atomes de cycle.

3. Complexe selon l'une quelconque des revendications 1 à 2,
les radicaux R¹ et R³ représentant chacun un radical-(C₆-C₂₀)-hétéroaryle comportant au moins six atomes de cycle.

4. Complexe selon l'une quelconque des revendications 1 à 3,
les radicaux R¹ et R³ représentant chacun 2-pyridinyle.

5. Complexe selon l'une quelconque des revendications 1 à 4,
R² et R⁴ représentant -(C₁-C₁₂)-alkyle.

6. Complexe selon l'une quelconque des revendications 1 à 5,
R² et R⁴ représentant tert-butyle.

7. Complexe selon l'une quelconque des revendications 1 à 6,
le composé (I) présentant la structure (1) :

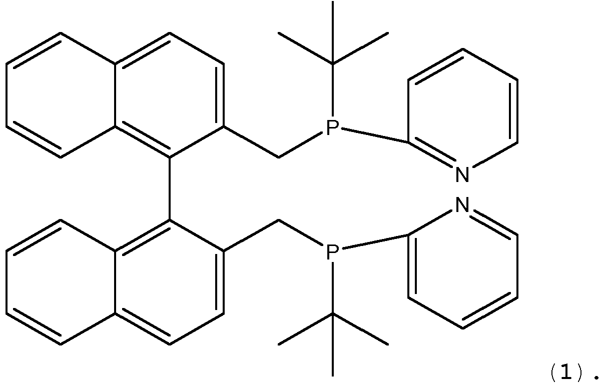

8. Procédé comprenant les étapes de procédé :
a) chargement d'un composé éthyléniquement insaturé ;
b) ajout d'un complexe selon l'une quelconque des revendications 1 à 7, ou d'un composé de formule (I) 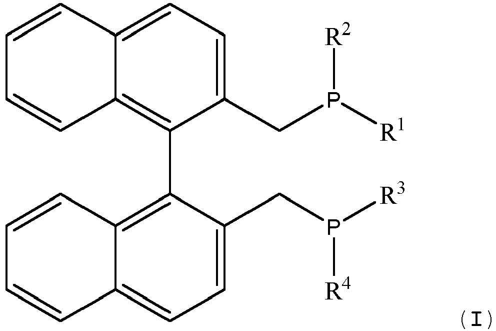
R¹, R², R³, R⁴ étant chacun indépendamment choisis parmi -(C₁-C₁₂)-alkyle, -(C₆-C₂₀)-hétéroaryle et
d'une substance qui comprend du Pt ;
c) ajout d'un acide ;
d) introduction de CO ;
e) chauffage du mélange réactionnel de a) à d), le composé éthyléniquement insaturé étant transformé en un acide carboxylique.

9. Procédé selon la revendication 8,
le composé éthyléniquement insaturé étant choisi parmi : éthène, propène, 1-butène, *cis-* et/ou trans-2-butène, isobutène, 1,3-butadiène, 1-pentène, *cis-* et/ou *trans-2-*pentène, 2-méthyl-1-butène, 3-méthyl-1-butène, 2-méthyl-2-butène, hexène, tétraméthyléthylène, heptène, 1-octène, 2-octène, di-n-butène, ou des mélanges correspondants.

10. Procédé selon l'une quelconque des revendications 8 et 9,
l'acide dans l'étape de procédé c) étant choisi parmi : acide acétique, acide perchlorique, acide sulfurique, acide phosphorique, acide méthylphosphonique, acide méthanesulfonique, acide trifluorométhanesulfonique, acide tert-butanesulfonique, acide p-toluènesulfonique, acide 2-hydroxypropane-2-sulfonique, acide 2,4,6-triméthylbenzènesulfonique, acide dodécylsulfonique, acide camphorsulfonique.

11. Procédé selon l'une quelconque des revendications 8 à 10,
l'acide dans l'étape de procédé c) étant l'acide acétique.

12. Procédé selon l'une quelconque des revendications 8 à 11,
la substance qui comprend du Pt étant choisie parmi : dichlorure de platine (PtCl₂), acétylacétonate de platine(II) [Pt(acac)₂], acétate de platine(II) [Pt(OAc)₂], dichloro(1,5-cyclooctadiène)platine(II) [Pt(cod)₂Cl₂], bis(dibenzylidèneacétone)platine [Pt(dba)₂], bis(acétonitrile)dichloroplatine(II) [Pt(CH₃CN)₂Cl₂], dichlorure de (cinnamyl)platine [Pt(cinnamyl)Cl₂].

13. Procédé selon l'une quelconque des revendications 8 à 12,
la substance qui comprend du Pt étant choisie parmi : dichlorure de platine (PtCl₂), acétylacétonate de platine (II) [Pt(acac)₂], acétate de platine (II) [Pt(OAc)₂].
